# FASCICULE DE BREVET EUROPEEN

(11) **EP 1 119 600 B1**
(45) Date de publication et mention de la délivrance du brevet: **20.07.2005**
(21) Numéro de dépôt: 99970111.3
(22) Date de dépôt: 06.10.1999
(51) Int. Cl.: C11C 3/10, C07C 67/03, A23J 3/14, C07C 31/22

(54) **PROCEDE DE FABRICATION D'ESTERS D'ACIDES GRAS, DE FARINE DE PROTEINES, DE FIBRES ET DE GLYCEROL PAR TRANSESTERIFICATION DIRECTE DE SOURCES DE MATIERES GRASSES**
VERFAHREN ZUR HERSTELLUNG VON FETTSÄUREESTERN, PROTEINMEHL, FASERN UND GLYCEROL DURCH DIE DIREKTE UMESTERUNG VON FETTQUELLEN
METHOD FOR PRODUCING ESTERS OF FATTY ACIDS, PROTEIN FLOUR, FIBRES AND GLYCEROL BY DIRECT TRANSESTERIFICATION OF FATS SOURCES

(30) Priorité: 06.10.1998 FR 9812484
(43) Date de publication de la demande: 01.08.2001
(73) Titulaire: NICKERSON INTERNATIONAL RESEARCH G.E.I.E., 63720 Chappes (FR); LIMACLUB S.A.S, 63720 Chappes (FR); UCS DEVELOPPEMENT, 78490 Galluis (FR); Société Coopérative Agricole E MC2, 55100 Bras-sur-Meuse (FR); VEGAM, 35134 Coesmes (FR)
(72) Inventeur: HOANG, Lê, Chiên, F-86340 Nouaille Maupertuis (FR)
(74) Mandataire: Nevant, Marc
(86) Numéro de dépôt international: PCT/FR1999/002391
(87) Numéro de publication internationale: WO 2000/020540

(56) Documents cités:
- WO-A1-96/31579
- WO-A1-96/33861
- WO-A1-97/38069
- US-A- 3 816 389
- CHEMICAL ABSTRACTS, vol. 123, no. 22, 27 novembre 1995 (1995-11-27) Columbus, Ohio, US; abstract no. 290360q, J. TICHY ET AL.: "Process of obtaining glycerol in the manufacture of biodiesel fuel from rape oil" page 263; colonne 2; XP002106481 & CZ 278 907 B6 (TICHY JOSEF DOC ING DRSC) 17 Août 1994
- KEVIN J. HARRINGTON ET AL.: "A comparison of conventional and in situ methods of transesterification of seed oil from a series of sunflower cultivars" JOURNAL OF THE AMERICAN OIL CHEMISTS' SOCIETY., vol. 62, no. 6, 1985, pages 1009-1013, XP002106480 CHAMPAIGN US

## Description

La présente invention concerne le domaine de l'oléochimie et de l'agro-alimentaire. Notamment, l'invention concerne un procédé de fractionnement de sources de matières grasses, notamment de graines d'oléagineux, pour obtenir des esters d'acides gras, des farines de protéines, des fibres et du glycérol.

Les graines d'oléagineux contiennent de l'huile (lipides), des protéines et des carbohydrates (celluloses, sucres). Ces graines sont actuellement destinées essentiellement à la fabrication d'huile alimentaire et de tourteaux (aliments du bétail). On fabrique les huiles brutes et les tourteaux par trituration (pressage et extraction à l'hexane des graines), ce qui entraîne que les protéines sont fortement mélangées avec des fibres dans les tourteaux. Les huiles brutes sont ensuite raffinées (démucilagination, neutralisation, décirage, décoloration, désodorisation) pour obtenir des huiles alimentaires. On trouve donc difficilement sur le marché des farines de protéines d'oléagineux de qualité alimentaire.

Quant aux esters d'acides gras d'oléagineux, ils sont fabriqués industriellement soit par transestérification des huiles semi-raffinées par du méthanol (fabrication d'esters méthyliques), soit par estérification des acides gras par un alcool comme l'éthanol, le propanol-1, le propanol-2, le butanol, ou l'isobutanol.

Ces procédés demandent de nombreuses étapes de transformation physico-chimique, ce qui entraîne des pertes importantes de matières d'ou notamment un coût de fabrication élevé. De plus, comme les protéines sont fortement mélangées avec des fibres dans les tourteaux, elles n'ont de ce fait qu'une seule valorisation possible, comme aliments du bétail.

La demande de brevet intemationale WO 97/38069 décrit un procédé de fabrication d'esters d'acides gras à partir de graines de tournesol oléique, dans lequel les graines sont directement soumises à une transformation chimique au moins partielle de transestérification en présence d'un réactif de transestérification constitué par un alcool et d'un catalyseur de transestérification, où la transformation chimique de transestérification est mise en oeuvre en continu dans un dispositif bi-vis de façon à faire subir simultanément aux graines un traitement mécanique de cisaillement, l'alcool et le catalyseur étant injectés dans le dispositif bi-vis au voisinage de l'entrée des graines avec un ajustement de la quantité d'alcool par rapport aux graines tel que la proportion d'alcool soit sensiblement comprise entre 0,3P et 3P, où P est la proportion d'alcool correspondant à la stoechiométrie de la réaction de transestérification : glycéride + alcool → ester + glycérol. Le dispositif bi-vis utilisé dans ce procédé comprend, d'amont en aval, une zone dotée de malaxeurs de cisaillement à l'exclusion de malaxeurs de compression axiale, une zone de soutirage de la phase liquide à travers un filtre et une zone terminale dotée de moyens de compression axiale des matières solides avant leur sortie.

Toutefois, ce procédé possède les inconvénients suivants :
- le traitement mécanique au moyen de la bi-vis ne permet pas de valoriser la partie solide de l'extrudat (farine de protéines ; fibres) ;
- les conditions de température et pression élevées utilisées par la suite pour fractionner l'extrudat, dénaturent considérablement les protéines ;
- il n'est pas possible de récupérer le catalyseur, car celui-ci est mélangé avec le résidu solide ;
- le rendement de pressage en esters d'acides gras est faible puisqu'il ne dépasse pas 43 % en masse.

Ce procédé est donc incompatible avec le fractionnement complet des graines oléagineuses.

La présente invention se propose de remédier à ces inconvénients au moyen d'un procédé qui permet le fractionnement direct de sources de matières grasses, notamment des graines oléagineuses.

Un objet de l'invention est donc de diminuer les coûts de fabrication des esters d'acides gras tout en valorisant les protéines de ces sources de matières grasses telles que les graines oléagineuses.

Un autre objet de l'invention consiste à obtenir notamment des esters d'acides gras et des farines de protéines à partir de ces sources de matières grasses telles que les graines oléagineuses avec un excellent rendement.

Un autre objet de l'invention est de conserver les propriétés des protéines de ces sources de matières grasses telles que les graines olégineuses.

Un autre objet de l'invention consiste à fournir un procédé qui permette de récupérer le catalyseur utilisé.

Ces objets sont atteints par le procédé de l'invention qui permet d'obtenir des esters d'acides gras, du glycérol et éventuellement des farines de protéines et/ou des fibres protéinées à partir de sources de matières grasses, notamment de graines entières oléagineuses. Ce procédé comprend les étapes suivantes :
a) la transestérification d'une source de matières grasses, notamment de graines oléagineuse, en milieu alcoolique en présence d'un catalyseur basique ;
b) la séparation des deux phases résultant de la transestérification ;
c) la récupération éventuelle de la farine de protéines à partir de la phase supérieure ;
d) la neutralisation de la phase supérieure restante avec un acide pour précipiter le catalyseur ; et
e) la distillation de la phase neutralisée et la décantation du distillat pour séparer les esters d'acides gras du glycérol.

Au sens de l'invention, on entend par "source de matières grasses" notamment des graines (entières) oléagineuses, des tourteaux gras oléagineux ou tout autre source végétale ou animale de matières grasses.

Par "graines oléagineuses" (ou graines d'oléagineux) on entend toute partie de plante, telle que par exemple graine, fleur ou fruit, pépin ou noyau, riche en lipides et éventuellement en glycérides et/ou en protéines.

Dans le cadre de l'invention, on peut notamment utiliser du colza, de l'arachide, du ricin, du sésame, de l'olivier, du toumesol, du carthame, du soja, du lupin de la cameline, du coton, du son de riz, du palme, du coco (coprah), des pépins de raisin ou de la bourrache, le colza (notamment le colza érucique), le tournesol et la cameline étant particulièrement avantageux.

Par "graines entières", on entend des graines broyées, éventuellement séchées, contenant toutes les substances naturellement présentes dans les graines.

Par "farine de protéines", on entend une farine qui comprend au moins 50 % de protéines et avantageusement au plus 20 % de fibres cellulosiques.

Par "fibres protéinées", on entend des fibres qui contiennent au moins 5 % de fibres cellulosiques et moins de 50 % de protéines. Par exemple, les fibres protéinées de colza comprennent environ 40 % de fibres cellulosiques et de 20 à 35 % de protéines.

Dans le procédé conforme à l'invention, la source de matières grasses, en particulier les graines d'oléagineux, est (sont) de préférence séchée(s) jusqu'à un taux d'humidité résiduel compris dans la gamme de 0 à 15 %, de préférence de 2 à 10 %, de préférence encore de 2 à 5 %, en masse, et broyée(s) avant la transestérification proprement dite. La température de séchage varie généralement de 40 à 105° C, et est de préférence comprise entre 50 et 70°C.

Eventuellement, selon la nature des graines, celles-ci peuvent être pré-broyées avant d'être séchées.

La transestérification s'effectue en milieu alcoolique en présence d'un catalyseur basique. La réaction se déroule à une température de 10 à 140°C. de préférence de 20 à 60°C, pendant environ 10 minutes à 6 heures, de préférence 30 minute à 2 heures, sous agitation soutenue.

Le milieu alcoolique comprend un ou plusieurs alcools en C₁-C₁₈, de préférence C₁-C₆, ou un mélange d'un alcool en C₁-C₁₈, de préférence C₁-C₆ et d'un autre solvant organique comme par exemple une cétone telle que l'acétone ou un hydrocarbure aliphatique tel que l'hexane ou l'heptane. Avantageusement, le milieu alcoolique comprend de l'éthanol ou un mélange d'éthanol et d'un autre solvant organique tel que mentionné ci-dessus.

Le catalyseur basique utilisé dans le procédé de l'invention est de préférence anhydre et peut être choisi par exemple parmi la soude, la potasse, le carbonate ou l'hydrogénocarbonate de sodium ou de potassium, le carbonate de sodium ou de potassium, le méthylate de sodium ou de potassium, l'éthylate de sodium ou de potassium ou bien encore un catalyseur aminé comme par exemple la diéthanolamine, le 1,6-diaminohexane, la 1,1,3,3-tétraméthylguanidine, la triéthylamine, la triéthanolamine ou le 2-aminoéthanol. Avantageusement, on utilise de la soude, de la potasse ou de l'éthylate de sodium ou de potassium.

Afin d'optimiser le rendement de la réaction de transestérification, il est souhaitable d'opérer avec un rapport massique catalyseur basique/alcool (milieu alcoolique)/graines compris dans la gamme 0,005 à 0,1/0,5 à 1,5/1.

Une fois la réaction terminée, le mélange réactionnel comprend deux phases qui sont séparées après décantation.

La phase inférieure est filtrée et le produit solide (contenant éventuellement les fibres protéinées) filtré est lavé à l'alcool (ou avec le milieu alcoolique), puis séché, de préférence par distillation, afin de récupérer le cas échéant les fibres sèches et avantageusement l'alcool.

La teneur en protéines des fibres récupérées peut être mesurée par la norme NFV18-120 (méthode Dumas).

La phase supérieure se compose d'une suspension alcoolique comprenant éventuellement la farine de protéines, les esters d'acide gras, le glycérol et le catalyseur. Cette phase est filtrée ou centrifugée. Le produit filtré ou le culot de centrifugation est ensuite rincé à l'alcool (ou avec le milieu alcoolique) puis séché, de préférence par distillation, afin d'obtenir le cas échéant la farine de protéines sèche.

Le filtrat résultant de ces opérations (c'est-à-dire le filtrat ou sumageant initial et le filtrat de rinçage) comprend donc les esters d'acides gras, le glycérol et le catalyseur.

Selon un mode de réalisation préféré de l'invention, le filtrat résultant de la récupération des fibres, est le cas échéant joint au filtrat résultant de la récupération de la farine de protéines.

On neutralise le catalyseur dans le filtrat (les filtrats joints) par un acide afin de précipiter le catalyseur basique sous forme de sel. A titre d'exemple d'acide approprié à cette fin, on peut citer notamment l'acide chlorhydrique, l'acide phosphorique, l'acide citrique ou l'acide acétique.

Après filtration pour éliminer le sel du catalyseur précipité, la solution alcoolique neutralisée est distillée sous vide à une température généralement comprise entre 40 et 140°C pour obtenir d'une part l'alcool, d'autre part les esters d'acides gras et le glycérol bruts, qui sont ensuite séparés par décantation.

Les esters d'acides gras sont ensuite purifiés par lavage à l'eau puis séchage, de préférence par distillation.

Le procédé conforme à l'invention, aussi appelé procédé MULTIVAL-1, permet de réduire les coûts de fabrication des esters d'acides gras et de valoriser les protéines de différentes sources de matières grasses, en particulier des oléagineux. Il présente également les avantages suivants :
- il permet de valoriser l'intégralité de la graine oléagineuse ;
- il peut être mis en oeuvre à basse température (< 60°C) et uniquement avec de l'alcool comme solvant de transestérification ;
- le catalyseur basique est récupéré (sous forme de sel);
- les rendements en esters d'acides gras, en farine de protéines et en fibres protéinées sont excellents, en particulier le rendement en esters d'acides gras est supérieur à 90 % ;
- il peut être mis en oeuvre en continu, en semi-continu ou en discontinu ;
- il peut être réalisé avec des installations standards de l'industrie chimique.

Ainsi, selon un autre aspect, l'invention concerne les esters d'acides gras, notamment d'acide érucique, les farines de protéines, les fibres protéinées et le glycérol, susceptibles d'être obtenus par le procédé décrit ci-dessus.

L'invention et ses avantages, seront mieux compris à la lecture des exemples ci-après donnés à titre purement illustratif.

### Exemple 1 (CF 170498)

On sèche des graines de colza érucique nettoyées dans une étuve ventilée à 60°C pendant 20 h pour obtenir un taux d'humidité final de 2,2 % massique.

On broie 400 g de graines sèches de colza par un mixeur de marque "Robot coupe" de 5 litres de capacité pendant 4 minutes.

On introduit la totalité du mélange de graines broyées dans un réacteur d'un litre à double enveloppe muni d'un agitateur et d'un réfrigérant à reflux. On ajoute dans le réacteur 600 g d'éthanol à 99 % et 2,82 g de NaOH à 97 %. On élève la température dans le réacteur jusqu'à 40°C par un bain marie avec une agitation soutenue. La transestérification dure dans ces conditions pendant 2 heures.

On ajoute dans le réacteur 200 g d'éthanol à 99 % et on agite pendant 5 minutes puis on laisse décanter les fibres pendant 5 minutes. On filtre sur un filtre presse de la marque Bioblock® (réf. : G10867) la phase supérieure contenant la farine de protéines suspendue dans l'éthanol. On répète les opérations précédentes 2 fois. On rince la farine sur le filtre avec deux fois 200 g d'éthanol. La farine de protéines est ensuite séchée à 60°C par distillation sous vide (40 mmHg) pour éliminer l'éthanol.

On filtre ensuite les fibres (phase inférieure) sur le même filtre presse et on rince les fibres par 2 fois 200 g d'éthanol. On sèche les fibres à 60°C par distillation sous vide (40 mmHg) pour éliminer l'éthanol.

Les solutions alcooliques issues des filtrations de la farine et des fibres sont mélangées et neutralisées par 3 g de H₃PO₄ à 75 %. On laisse décanter les flocons de sel de phosphate d'alcalin pendant au moins une heure. On élimine ensuite ces flocons par filtration (sur filtre presse).

On distille la solution alcoolique neutralisée à 60°C sous vide (40 mmHg) pour éliminer l'éthanol et obtenir les esters bruts (composés majoritairement d'esters éthyliques avec des traces de mono-, di- et triglycérides) et la glycérine brute La glycérine brute et les esters bruts sont séparés par décantation (au moins 10 minutes de décantation).

On lave les esters bruts par de l'eau à 95°C (0,5 volume d'eau pour 1 volume d'ester pour chaque lavage, 3 lavages) pour éliminer les traces de phosphore et de sel de phosphate d'alcalin. On distille ensuite les esters lavés à 95°C sous vide (40 mmHg) pour obtenir des esters purifiés. La quantité de matières obtenue est la suivante :

| **Matières** | **Quantité** |
|---|---|
| Fibres protéinées | 221.5 g |
| Farine de protéines | 61,9 g |
| Glycérine brute dont | 14,8 g |
| Glycérol | > 90 % |
| Esters éthyliques dont | 113,3 g |
| Esters éthyliques | 97,5 % |
| Monoglycérides | < 1,5 % |
| Diglycérides | < 0,5 % |
| Triglycérides | < 0,5 % |

### Exemple 2 (VL 240398)

On procède de la même manière qu'à l'exemple 1, à l'exception du fait que l'on remplace les 2,82 g de NaOH à 97 % par 4,5 g de KOH anhydre à 85 % comme catalyseur de transestérification. La quantité de matières obtenue est la suivante :

| **Matières** | **Quantité** |
|---|---|
| Fibres protéinées | 203,5 g |
| Farine de protéines | 67,9 g |
| Glycérine brute dont | 13,4 g |
| Glycérol | > 90 % |
| Esters éthyliques dont | 102,3 g |
| Esters éthyliques | 98 % |
| Monoglycérides | < 1,0 % |
| Diglycérides | < 0,5 % |
| Triglycérides | < 0,5 % |

### Exemple 3 (CF 100498)

On procède de la même manière qu'à l'exemple 1, à l'exception du fait que l'on remplace les 2,82 g de NaOH à 97 % par 4,84 g de C₂H₅ONa à 96 % comme catalyseur de transestérification. La quantité de matières obtenue est la suivante :

| **Matières** | **Quantité** |
|---|---|
| Fibres protéinées | 223,9 g |
| Farine de protéines | 68,7 g |
| Glycérine brute dont | 14,1 g |
| Glycérol | > 90 % |
| Esters éthyliques dont | 108,1 g |
| Esters éthyliques | 98 % |
| Monoglycérides | < 1,0 % |
| Diglycérides | < 0,5 % |
| Triglycérides | < 0,5 % |

### Exemple 4 (CF 060498)

On procède de la même manière qu'à l'exemple 1, à l'exception du fait que l'on remplace les 2,82 g de NaOH à 97 % par 6,04 g de C₂H₅OK à 95 % comme catalyseur de transestérification. La quantité de matières obtenue est la suivante :

| **Matières** | **Quantité** |
|---|---|
| Fibres protéinées | 193,3 g |
| Farine de protéines | 94 g |
| Glycérine brute dont | 14,7 g |
| Glycérol | > 90 % |
| Esters éthyliques dont | 112,8 g |
| Esters éthyliques | 98,5 % |
| Monoglycérides | < 1,0 % |
| Diglycérides | < 0,5 % |
| Triglycérides | 0 % |

### Exemple 5 (CF 220498)

On procède de la même manière qu'à l'exemple 4, à l'exception du fait que l'on utilise 400 g de graines broyées dans 400 g d'éthanol à 99 % et qu'on ajoute 200 g d'éthanol pour la transestérification. La quantité de matières obtenue est la suivante :

| **Matières** | **Quantité** |
|---|---|
| Fibres protéinées | 219 g |
| Farine de protéines | 70,1 g |
| Glycérine brute dont | 15,4 g |
| Glycérol | > 90 % |
| Esters éthyliques dont | 117,8 g |
| Esters éthyliques | 98,6 % |
| Monoglycérides | < 1,0 % |
| Diglycérides | < 0,5 % |
| Triglycérides | < 0,5 % |

Le rendement dans le cadre de cet exemple est avantageusement augmenté.

### Exemple 6 (CF 280498)

On procède de la même manière qu'à l'exemple 5, à l'exception du fait que l'on utilise 4,54 g de C₂H₅OK à 95 % comme catalyseur et 2,25 g de H₃PO₄ pour la neutralisation. La quantité de matières obtenue est la suivante :

| **Matières** | **Quantité** |
|---|---|
| Fibres protéinées | 194,5 g |
| Farine de protéines | 82,8 g |
| Glycérine brute dont | 15,6 g |
| Glycérol | > 90 % |
| Esters éthyliques dont | 119,5 g |
| Esters éthyliques | 99,5 % |
| Monoglycérides | < 0,5 % |
| Diglycérides | 0 % |
| Triglycérides | 0 % |

### Exemple 7 (CF 060598)

On procède de la même manière qu'à l'exemple 6, à l'exception du fait que l'on effectue la transestérification à 30°C au lieu de 40°C. La quantité de matières obtenue est la suivante :

| **Matières** | **Quantité** |
|---|---|
| Fibres protéinées | 210 g |
| Farine de protéines | 84,1 g |
| Glycénne brute dont | 15 g |
| Glycérol | > 90 % |
| Esters éthyliques dont | 114,7 g |
| Esters éthyliques | 99,0 % |
| Monoglycérides | 0,6 % |
| Diglycérides | 0 % |
| Triglycérides | 0 % |

### Exemple 8 (CF 250598)

On procède de la même manière qu'à l'exemple 6, à l'exception du fait que l'on remplace l'éthanol à 99 % par un mélange éthanol 99 %/n-hexane 95 % avec un rapport volumique n-hexane/éthanol de 0,2/1 dans toutes les opérations. La quantité de matières obtenue est la suivante :

| **Matières** | **Quantité** |
|---|---|
| Fibres protéinées | 178,8 g |
| Farine de protéines | 88 g |
| Glycérine brute dont | 17,3 g |
| Glycérol | > 90 % |
| Esters éthyliques dont | 132,5 g |
| Esters éthyliques | 98,9 % |
| Monoglycérides | 0,8% |
| Diglycérides | 0,3 % |
| Triglycérides | 0 % |

### Exemple 9 (CF 270598)

On procède de la même manière qu'à l'exemple 6, à l'exception du fait que l'on remplace l'éthanol à 99 % par un mélange éthanol 99 %/n-heptane 98 % avec un rapport volumique n-heptane/éthanol de 0,2/1 dans toutes les opérations. La quantité de matières obtenue est la suivante :

| **Matières** | **Quantité** |
|---|---|
| Fibres protéinées | 180,5 g |
| Farine de protéines | 81 g |
| Glycérine brute dont | 18,2 g |
| Glycérol | > 90 % |
| Esters éthyliques dont | 139,1 g |
| Esters éthyliques | 98,2 % |
| Monoglycérides | 0,8 % |
| Diglycérides | 0,5 % |
| Triglycérides | 0.5 % |

Il apparaît que l'utilisation d'un co-solvant augmente avantageusement le rendement.

### Exemple 10 (CF 020698)

On procède de la même manière qu'à l'exemple 6, à l'exception du fait que l'on remplace l'éthanol à 99 % par un mélange éthanol 99 %/acétone 95 % avec un rapport volumique acétone/éthanol de 0,2/1 dans toutes les opérations. La quantité de matières obtenue est la suivante :

| **Matières** | **Quantité** |
|---|---|
| Fibres protéinées | 198,4 g |
| Farine de protéines | 78,1 g |
| Glycérine brute dont | 17,8 g |
| Glycérol | > 90 % |
| Esters éthyliques dont | 136,7 g |
| Esters éthyliques | 98,8 % |
| Monoglycérides | 0,7 % |
| Diglycérides | 0,3 % |
| Triglycérides | 0 % |

### Exemple 11 (LC 030196A)

On effectue les mêmes opérations que dans le protocole général de l'exemple 1 mais on remplace les graines de colza érucique par 100 g de graines de tournesol classique (taux d'humidité résiduel : 9,2 % massique) et on utilise 4 g de K₂CO₃ à 99 % comme catalyseur et 2,65 g de H₃PO₄ pour la neutralisation. La transestérification a été réalisée à 78°C. La quantité de matières obtenue est la suivante :

| **Matières** | **Quantité** |
|---|---|
| Fibres protéinées | 41 g |
| Farine de protéines | 21 g |
| Glycérine brute dont | 5 g |
| Glycérol | > 90 % |
| Esters éthyliques dont | 36,2 g |
| Esters éthyliques | 13,03 % |
| Monoglycérides | 72,0 % |
| Diglycérides | 11.76 % |
| Triglycérides | 2,95 % |

| Composition d'acides gras des esters obtenus à partir de tournesol classique (Méthode de mesure : NF ISO 5508) | |
|---|---|
| **Acide gras** | **% massique** |
| acide palmitique (C16:0) | 5,5 - 6.0 |
| acide stéarique (C18:0) | 5 - 5,5 |
| acide oléique (C18:1) | 25 - 27 |
| acide linoléique (C18:2) | 65 - 70 |
| acide linolénique (C18:3) | 0 |
| acide arachidique (C20:0) | < 0,5 |
| acide éicosénoïque (C20:1) | 0 |
| acide béhénique (C22:0) | < 1 |
| acide érucique (C22:1) | 0 |

### Exemple 12 (LC 091096)

On effectue les mêmes opérations que dans l'exemple 11 mais on utilise 385 g de graines de tournesol oléique, 15 g de KOH à 85 % anhydre comme catalyseur et 10 g de H₃PO₄ pour la neutralisation. La quantité (non séchée) de matières obtenue est la suivante :

| **Matières** | **Quantité** |
|---|---|
| Fibres protéinées | 130,8 g |
| Farine de protéines | 71 g |
| Glycérine brute dont | 14,9 g |
| Glycérol | > 90 % |
| Esters éthyliques dont | 169,2 g |
| Esters éthyliques | 53,75 % |
| Monoglycérides | 38,8 % |
| Diglycérides | 2,41 % |
| Triglycérides | 5.1 % |

| Composition d'acides gras des esters obtenus à partir de tournesol oléique (Méthode de mesure : NF ISO 5508) | |
|---|---|
| **Acide gras** | **% massique** |
| acide palmitique (C16:0) | 3,0 - 4,5 |
| acide stéarique (C18:0) | 4,5 - 5,0 |
| acide oléique (C18:1) | 83 - 85 |
| acide linoléique (C18:2) | 8,5 - 10 |
| acide linolénique (C18:3) | 0 |
| acide arachidique (C20:0) | < 0,5 |
| acide éicosénoïque (C20:1) | 0 |
| acide béhénique (C22:0) | < 1 |
| acide érucique (C22:1) | 0 |

### Exemple 13 (LC 011096)

On effectue les mêmes opérations que dans le protocole général de l'exemple 1 mais on remplace les graines de colza érucique par 303 g de son de riz (taux d'humidité résiduel : 6 % massique) et on utilise 12.3 g de KOH à 85 % anhydre comme catalyseur et 14,5 g de H₃PO₄ pour la neutralisation. La transestérification a été réalisée à 78°C. La quantité de matières obtenue est la suivante :

| **Matières** | **Quantité** |
|---|---|
| Fibres protéinées | 200 g |
| Farine de protéines | 67 g |
| Glycérine brute dont | 5 g |
| Glycérol | > 90 % |
| Esters éthyliques dont | 30 g |
| Esters éthyliques | 76,0 % |
| Monoglycérides | 8,62 % |
| Diglycéndes | 5,85 % |
| Triglycérides | 4,2 % |

| Composition d'acides gras des esters obtenus à partir de son de riz (Rice Bran) (Méthode de mesure : NF ISO 5508) | |
|---|---|
| **Acide gras** | **% massique** |
| acide palmitique (C16:0) | 17 |
| acide stéarique (C18:0) | 2 |
| acide oléique (C18:1) | 40 |
| acide linoléique (C18.2) | 34 |
| acide linolénique (C18:3) | 1 |

### Exemple 14 (LC 111096A)

On effectue les mêmes opérations que dans l'exemple 11 mais avec 376 g de graines de tournesol oléique, 15 g de NaOH à 97 % comme catalyseur et 16 g de H₃PO₄ pour la neutralisation. La quantité de matières obtenue est la suivante :

| **Matières** | **Quantité** |
|---|---|
| Fibres protéinées | 127,7 g |
| Farine de protéines | 83 g |
| Glycérine brute dont | 19,5 g |
| Glycérol | > 90 % |
| Esters éthyliques dont | 151,8 g |
| Esters éthyliques | 32,76 % |
| Monoglycérides | 63,43 % |
| Diglycérides | 2,25 % |
| Triglycérides | 0,95 % |

### Exemple 15 (LC 231096)

On effectue les mêmes opérations que dans le protocole général de l'exemple 1 mais avec 380 g de graines broyées de colza sans érucique (taux d'humidité résiduel : 9,3 % massique), 26 g de K₂CO₃ à 99 % comme catalyseur et 16,37 g de H₃PO₄ pour la neutralisation. La transestérification a été réalisée à 78°C. La quantité de matières obtenue est la suivante :

| **Matières** | **Quantité** |
|---|---|
| Fibres protéinées | 125 g |
| Farine de protéines | 84 g |
| Glycérine brute dont | 18,5 g |
| Glycérol | > 90 % |
| Esters éthyliques dont | 150 g |
| Esters éthyliques | 76,31 % |
| Monoglycéndes | 8,62 % |
| Diglycérides | 5,8 % |
| Triglycérides | 4,23% |

| Composition d'acides gras des esters obtenus à partir de colza sans érucique (Méthode de mesure : NF ISO 5508) | |
|---|---|
| **Acide gras** | **% massique** |
| acide palmitique (C16:0) | 3,9 - 4,2 |
| acide stéarique (C18:0) | 1,5 - 2.0 |
| acide oléique (C18:1) | 58 - 59 |
| acide linoléique (C18:2) | 18,8 - 26,3 |
| acide linolénique (C18:3) | 2,4 - 8,8 |
| acide arachidique (C20:0) | 0 |
| acide éicosénoïque (C20:1) | 1,3 - 1,4 |
| acide béhénique (C22:0) | < 0,5 |
| acide érucique (C22:1) | 0,4 - 0,5 |

### Exemple 16 (LC 071196)

On effectue les mêmes opérations que dans le protocole général de l'exemple 1 mais avec 400 g de graines de colza sans érucique broyées (taux d'humidité résiduel : 9,3 % massique), 10 g de KOH à 85 % comme catalyseur et 6,6 g de H₃PO₄ pour la neutralisation. La transestérification a été effectuée à 78°C. La quantité (non séchée) de matières obtenue est la suivante :

| **Matières** | **Quantité** |
|---|---|
| Fibres protéinées | 165.3 g |
| Farine de protéines | 121 g |
| Glycérine brute dont | 15 g |
| Glycérol | > 90 % |
| Esters éthyliques dont | 106,2 g |
| Esters éthyliques | 59,3 % |
| Monoglycérides | 17,2 % |
| Diglycérides | 4,5 % |
| Triglycérides | 20,7 % |

### Exemple 17 (LC 261196)

On effectue les mêmes opérations que dans l'exemple 16 mais on utilise 10,6 g de CH₃ONa à 99 % comme catalyseur et 8,5 g de H₃PO₄ pour la neutralisation. La quantité de matières obtenue est la suivante :

| **Matières** | **Quantité** |
|---|---|
| Fibres protéinées | 254,5 g |
| Farine de protéines | 25,3 g |
| Glycérine brute dont | 15 g |
| Glycérol | > 90 % |
| Esters éthyliques dont | 100,5 g |
| Esters éthyliques | 60 % |
| Monoglycérides | 23,6 % |
| Diglycérides | 6,4 % |
| Triglycérides | 9,8 % |

### Exemple 18 (LCH 300197)

On effectue les mêmes opérations que dans le protocole général de l'exemple 1, mais avec 400 g de graines de cameline (taux d'humidité résiduel : 8 % massique), 15 g de KOH à 85 % anhydre comme catalyseur et 8,8 g de H₃PO₄ pour la neutralisation. La transestérification a été réalisée à 78°C. La quantité de matières obtenue est la suivante :

| **Matières** | **Quantité** |
|---|---|
| Fibres protéinées | 256,4 g |
| Farine de protéines | 39 g |
| Glycérine brute dont | 10,2 g |
| Glycérol | > 90 % |
| Esters éthyliques dont | 103 g |
| Esters éthyliques | 68,1 % |
| Monoglycérides | 29,8 % |
| Diglycérides | 1,7 % |
| Triglycérides | 0,4 % |

| Composition d'acides gras des esters obtenus à partir de cameline (Méthode de mesure : NF ISO 5508) | |
|---|---|
| **Acide gras** | **% massique** |
| acide myristique (C14:0) | < 0,1 |
| acide palmitique (C16:0) | 4 - 7 |
| acide stéarique (C18:0) | 2,4 - 2,6 |
| acide oléique (C18:1) | 19,0 - 20,0 |
| acide linoléique (C18:2) | 18,0 - 18,5 |
| acide linolénique (C18:3 n-6) | 30,0 - 30,4 |
| acide arachidique (C20:0) | 1,4 - 1,6 |
| acide éicosénoïque (C20:1) | 15,0 - 15,3 |
| (C20:2) | 1,5 - 1,6 |
| (C20:3) | 1,0 - 1,2 |
| acide béhénique (C22:0) | < 0,5 |
| acide érucique (C22:1) | 3,2 - 3,5 |
| (C24:0) | < 0,2 |
| (C24:1) | < 0,5 |

### Exemple 19 (LCH 140197)

On effectue les mêmes opérations que dans le protocole général de l'exemple 1 mais avec 400 g de graines de lupin doux (taux d'humidité résiduel : 6,5 % massique), 15 g de KOH à 85 % anhydre comme catalyseur et 8,75 g de H₃PO₄ pour la neutralisation. La transestérification a été réalisée à 78°C. La quantité de matières obtenue est la suivante :

| **Matières** | **Quantité** |
|---|---|
| Fibres protéinées | 286.6 |
| Farine de protéines | 70 g |
| Glycérine brute dont | 2,3 g |
| Glycérol | > 90 % |
| Esters éthyliques dont | 40,1 g |
| Esters éthyliques | 59,4 % |
| Monoglycérides | 38,7 % |
| Diglycérides | 1.5 % |
| Triglycérides | 0,4 % |

| Composition d'acides gras des esters obtenus à partir de lupin doux (blanc) (Méthode de mesure : NF ISO 5508) | |
|---|---|
| **Acides gras** | **% massique** |
| acide palmitique (C16:0) | 8,0 - 8,5 |
| acide stéarique (C18:0) | 2,0 - 2,2 |
| acide oléique (C18:1) | 50,9 - 51,5 |
| acide linoléique (C18:2) | 14,5 - 15,0 |
| acide linolénique (C18:3) | 10,5 - 11,0 |
| acide arachidique (C20:0) | 1,0 - 1,3 |
| acide éicosénoïque (C20:1) | 4,8 - 5,2 |
| acide béhénique (C22:0) | 4,5 - 5,5 |
| Acide érucique (C22:1) | 2,0 - 2,2 |

### Exemple 20 (LCH 190897)

On effectue les mêmes opérations que dans le protocole général de l'exemple 1 mais avec 400 g de graines de coton (taux d'humidité résiduel = 8 % massique), 15 g de KOH à 85 % anhydre comme catalyseur et 10 g de H₃PO₄ pour la neutralisation. La transestérification a été réalisée à 78°C. La quantité de matières obtenue est la suivante :

| **Matières** | **Quantité** |
|---|---|
| Fibres protéinées | 157 g |
| Farine de protéines | 147,7 g |
| Glycérine brute dont | 7 g |
| Glycérol | > 90 % |
| Esters éthyliques dont | 79,9 g |
| Esters éthyliques | 56 % |
| Monoglycérides | 38 % |
| Diglycérides | 2,6 % |
| Triglycérides | 3.4 % |

| Composition d'acides gras des esters obtenus à partir de coton (Méthode de mesure : NF ISO 5508) | |
|---|---|
| **Acide gras** | **% massique** |
| acide myristique (C14:0) | 1,0 |
| acide palmitique (C16:0) | 26,0 |
| acide stéarique (C18:0) | 2,0 |
| acide oléique (C18:1) | 19,0 |
| acide linoléique (C18:2) | 51 |
| acide linolénique (C18:3) | < 1 |

### Exemple 21 (LCH 300798)

On procède de la même manière qu'à l'exemple 6. à l'exception du fait que l'on utilise 20 kg de graines de colza érucique et 0,325 kg de KOH à 85 % anhydre comme catalyseur. La transestérification est réalisée dans un réacteur inox de 97 litres de capacité et thermostaté à 40°C. Les filtrations de la farine de protéines sont effectuées sur un filtre de poche de la marque Amafilter® (réf. : AF1-417T). La quantité de matières obtenue est la suivante :

| **Matières** | **Quantité** |
|---|---|
| Fibres protéinées | 5 kg |
| Farine de protéines | 6,2 kg |
| Glycérine brute dont | 0,74 kg |
| Glycérol | > 90 % |
| Esters éthyliques dont | 8,82 kg |
| Esters éthyliques | 99,0 % |
| Monoglycérides | < 0,5 % |
| Diglycérides | trace |
| Triglycérides | trace |

Le fractionnement des graines de colza érucique à l'échelle pilote (100 litres) permet d'obtenir de meilleurs résultats en rendement de farine de protéines (> 98 %) et d'esters éthyliques (> 96 %).

| Caractéristiques des esters d'acides gras obtenus à partir de colza érucique | | |
|---|---|---|
| **Caractéristiques physico-chimiques** | **Valeur** | **Méthode de mesure** |
| Teneur en matières volatiles, % | < 0,3 | NF T 60-201 |
| Masse volumique à 20°C, kg/l | 0,876 | NF ISO 6883 |
| Viscosité à 20°C, cPs | 9 - 10 | Rhéomètre |
| Indice d'iode - II | 95 - 96 | NF ISO 3961 |
| Indice d'acide, mg de KOH/g | < 0,2 | ISO 660-1983 |
| Indice de saponification, mg de KOH/g | 158 | NF ISO 3657 |
| Teneur en phosphore, ppm | 25 | AFNOR NF T 60-228 |
| Insaponifiable, % | 0,6 | NF T 60-205-2 |
| Glycérides partiels, % | < 0,5 | HPLC |
| Glycérol, % | Trace | HPLC |
| Taux de cendre, % | 0 | NF ISO 6884 |

| *Composition des acides gras* | | |
|---|---|---|
| **Acide gras** | **Teneur, %** | **Méthode de mesure** |
| acide palmitique (C16:0) | 2,8 | NF ISO 5508 |
| acide stéarique (C18:0) | 1,0 | |
| acide oléique (C18:1) | 14,4 | |
| acide linoléique (C18:2) | 12,5 | |
| acide linolénique (C18:3) | 7,7 | |
| acide arachidique (C20:0) | 0,7 | |
| acide éicosénoïque (C20:1) | 7,8 | |
| acide béhénique (C22:0) | 0,7 | |
| acide érucique (C22:1) | 49,7 | |
| (C24:0) | 0,6 | |
| (C24:1) | 0,6 | |
| (C26:0) | 1,0 | |
| (C26:1) | 0,5 | |

| Composition chimique de la glycérine brute obtenue à partir de colza érucique | | |
|---|---|---|
| **Substances chimiques** | **% massique** | **Méthode de mesure** |
| Matières volatiles | 9 | NF T 60-201 |
| Glycérol | > 90 | HPLC |
| Taux de cendre | 1,7 | NF ISO 6884 |

| Composition chimique de la farine de protéines obtenue à partir de colza érucique | | |
|---|---|---|
| **Substances chimiques** | **Teneur** | **Méthode de mesure** |
| Matières volatiles et eau, % | 7 - 8 | NF T 60-201 |
| Matières grasses, % | 3,8 | NF ISO 659 |
| Fibres totales, % | 18,59 | AOAC 985-29 (1990) |
| Glucosinolates, µmoles/g | 11,26 | CEE/HPLC |
| Protéines sur sec N*6.25, % | 53,20 | NF V 18-120 (Dumas) |
| Sucres totaux, % | 9,65 | CEE 1ère Directive |
| Tannins, % | 0 | Colorimétrie |

| Composition chimique des fibres protéinées obtenue à partir de colza érucique | | |
|---|---|---|
| **Substances chimiques** | **Teneur** | **Méthode de mesure** |
| Matières volatiles et eau, % | 8 - 9 | NF T 60-201 |
| Matières grasses, % | 3,1 | NF ISO 659 |
| Fibres totales, % | 42.77 | AOAC 985-29 (1990) |
| Glucosinolates, µmoles/g | - | CEE/HPLC |
| Protéines sur sec N*6.25, % | 33,50 | NF V 18-120 (Dumas) |
| Sucres totaux, % | 5,42 | CEE 1ère Directive |
| Tannins, % | - | Colorimétrie |

## Revendications

1. Procédé d'obtention d'esters d'acides gras, de glycérol et éventuellement de farines de protéines et/ou de fibres protéinées à partir d'une source de matières grasses, notamment de graines entières d'oléagineux, qui comprend les étapes suivantes :
a) la transestérification de la source de matières grasses, notamment des graines oléagineuses, en milieu alcoolique en présence d'un catalyseur basique ;
b) la séparation des deux phases résultant de la transestérification ;
c) la récupération éventuelle de la farine de protéines à partir de la phase supérieure ;
d) la neutralisation de la phase supérieure restante avec un acide pour précipiter le catalyseur ; et
e) la distillation de la phase neutralisée et la décantation du distillat pour séparer les esters d'acides gras du glycérol.

2. Procédé selon la revendication 1, dans lequel la source de matières grasses, notamment les graines oléagineuses, est (sont) séchée(s) jusqu'à un taux d'humidité résiduel compris entre O et 15 %, de préférence entre 2 et 10 % en masse, et broyée(s).

3. Procédé selon la revendication 2, dans lequel la source de matières grasses est pré-broyée avant le séchage.

4. Procédé selon l'une des revendications 1 à 3, dans lequel les fibres protéinées sont éventuellement récupérées à partir de la phase inférieure.

5. Procédé selon la revendication 4, dans lequel les fibres récupérées sont rincées avec le milieu alcoolique et le filtrat alcoolique est joint à la phase alcoolique avant la neutralisation de l'étape d).

6. Procédé selon l'une des revendications 1 à 5, dans lequel le milieu alcoolique comprend un ou plusieurs alcools en C₁-C₁₈, de préférence C₁-C₆, ou un mélange d'un alcool en C₁-C₁₈, de préférence C₁-C₆, et d'une cétone ou d'un hydrocarbure aliphatique.

7. Procédé selon la revendication 6, dans lequel le milieu alcoolique comprend de l'éthanol.

8. Procédé selon l'une des revendications 1 à 7, dans lequel le catalyseur basique utilisé pour la transestérification est un catalyseur anhydre.

9. Procédé selon la revendication 8, dans lequel le catalyseur est choisi parmi la soude, la potasse, l'éthylate de sodium ou l'éthylate de potassium.

10. Procédé selon l'une des revendications 1 à 9, dans lequel le rapport massique catalyseur/alcool/graines est compris dans la gamme 0,005 à 0,1/0,5 à 1,5/1.

11. Procédé selon l'une des revendications 1 à 10, dans lequel la graine d'oléagineux est une graine de colza, de tournesol ou de cameline.

12. Farine de protéines susceptible d'être obtenue par le procédé selon l'une des revendications 1 à 11, qui comprend au moins 50 % de protéines et au plus 20 % de fibres cellulosiques.

13. Fibres protéinées susceptibles d'être obtenues par le procédé selon l'une des revendications 1 à 11, qui comprennent au moins 5 % de fibres cellulosiques et moins de 50 % de protéines.

## Patentansprüche

1. Verfahren zum Erhalt Von Fettsäureestern, Glycerin und gegebenenfalls Proteinmehl und/oder Proteinfasern aus einer Fettquelle, insbesondere aus ganzen Kernen bzw. Körnern von Ölpflanzen, umfassend die folgenden Schritte:
a) Umestem der Fettquelle, insbesondere der Kerne bzw. der Körner von Ölpflanzen, in alkoholischem Medium in Gegenwart eines basischen Katalysators;
b) Trennen der beiden aus der Umesterung resultierenden Phasen;
c) gegebenenfalls Gewinnen des Proteinmehls aus der oberen Phase;
d) Neutralisation der verbleibenden oberen Phase mit einer Säure, um den Katalysator auszufällen; und
e) Destillation der neutralisierten Phase und Dekantieren des Destillats, um die Fettsäureester von dem Glycerin zu trennen.

2. Verfahren nach Anspruch 1, wobei die Fettquelle, insbesondere die Kerne bzw. die Körner von Ölpflanzen, bis zu einem Restfeuchtigkeitsgehalt zwischen 0 und 15, vorzugsweise zwischen 2 und 10 Gew.-%, getrocknet und vermahlen wird/werden.

3. Verfahren nach Anspruch 2, wobei die Fettquelle vor dem Trocknen vorgemahlen wird.

4. Verfahren nach einem der Ansprüche 1 bis 3, wobei die Proteinfasern gegebenenfalls aus der unteren Phase gewonnen werden.

5. Verfahren nach Anspruch 4, wobei die gewonnenen Fasern mit dem alkoholischen Medium gespült werden und das alkoholische Filtrat der alkoholischen Phase vor der Neutralisation in Schritt d) zugegeben wird.

6. Verfahren nach einem der Ansprüche 1 bis 5, wobei das alkoholische Medium einen oder mehrere C₁-C₁₈-Alkohole, vorzugsweise C₁-C₆-Alkohole, oder ein Gemisch eines C₁-C₁₈-Alkohols, vorzugsweise eines C₁-C₆-Alkohols, und eines Ketons oder eines aliphatischen Kohlenwasserstoffs umfaßt.

7. Verfahren nach Anspruch 6, wobei das alkoholische Medium Ethanol umfaßt.

8. Verfahren nach einem der Ansprüche 1 bis 7, wobei der zur Umesterung verwendete basische Katalysator ein wasserfreier Katalysator ist.

9. Verfahren nach Anspruch 8, wobei der Katalysator aus Soda, Pottasche, Natriumethylat oder Kaliumethylat ausgewählt ist.

10. Verfahren nach einem der Ansprüche 1 bis 9, wobei das Gewichtsverhältnis Katalysator/Alkohol/Keme bzw. Körner im Bereich Von 0,005 bis 0,1/0,5 bis 1,5/1 liegt.

11. Verfahren nach einem der Ansprüche 1 bis 10, wobei der Kern bzw. das Kom der Ölpflanze ein Rapssamenkorn, Sonnenblumenkern oder Dotterblumensamenkorn ist.

12. Proteinmehl, erhältlich durch das Verfahren nach einem der Ansprüche 1 bis 11, umfassend mindestens 50% Proteine und höchstens 20% Cellulosefasern.

13. Proteinfasern, erhältlich durch das Verfahren nach einem der Ansprüche 1 bis 11, umfassend mindestens 5% Cellulosefasern und weniger als 50% Proteine.

## Claims

1. Method for obtaining esters of fatty acids, glycerol and optionally protein flours and/or protein fibres from a fat source, in particular from whole oleaginous seeds, which comprises the following steps:
a) transesterification of the fat source, oleaginous seeds in particular, in an alcohol medium in the presence of a basic catalyst;
b) separation of the two phases resulting from transesterification,
c) optional recovery of the protein flour from the upper phase;
d) neutralisation of the remaining upper phase with an acid to precipitate the catalyst; and
e) distillation of the neutralised phase and decanting of the distillate to separate the esters of fatty acids from the glycerol.

2. Method as in claim 1, in which the fat source, oleaginous seeds in particular, is dried to a residual moisture of between 0 and 15%, preferably between 2 and 10 % by weight, and ground.

3. Method as in claim 2, in which the fat source is pre-crushed before drying.

4. Method as in any of claims 1 to 3, in which the protein fibres are optionally recovered from the lower phase.

5. Method as in claim 4, in which the recovered fibres are rinsed in the alcohol medium and the alcohol filtrate is added to the alcohol phase before the neutralisation of step d) .

6. Method as in any of claims 1 to 5, in which the alcohol medium comprises one or more C₁-C₁₈ alcohols, preferably C₁-C₆, or a mixture of a C₁-C₁₈ alcohol preferably C₁-C₆ with a ketone or an aliphatic hydrocarbon.

7. Method as in claim 6, in which the alcohol medium comprises ethanol.

8. Method as in any of claims 1 to 7, in which the base catalyst used for transesterification is an anhydrous catalyst.

9. Method as in claim 8, in which the catalyst is chosen from among sodium hydroxide, potassium hydroxide, sodium ethylate or potassium ethylate.

10. Method as in any of claims 1 to 9, in which the catalyst/alcohol/grain weight ratio lies in the range of 0.005 to 0.1/0.5 to 1.5/1.

11. Method as in any of claims 1 to 10, in which the oleaginous seeds are rape, sunflower or camelina seeds.

12. Protein flour able to be obtained with the method as in any of claims 1 to 11, which comprises at least 50 % proteins and no more than 20 % cellulose fibres.

13. Protein fibres able to be obtained with the method as in any of claims 1 to 11, which comprise at least 5 % cellulose fibres and less than 50 % proteins.
